# EUROPÄISCHE PATENTSCHRIFT

(11) **EP 1 316 543 B1**
(45) Veröffentlichungstag und Bekanntmachung des Hinweises auf die Patenterteilung: **21.06.2006**
(21) Anmeldenummer: 02021495.3
(22) Anmeldetag: 26.09.2002
(51) Int. Cl.: C07C 227/00

(54) **Verfahren zur Herstellung von Aminocarbonsäuren**
Process for the preparation of aminocarboxylic acids
Procédé de préparation d'acides aminocarboxyliques

(30) Priorität: 29.11.2001 DE 10158537
(43) Veröffentlichungstag der Anmeldung: 04.06.2003
(73) Patentinhaber: Degussa AG, 40474 Düsseldorf (DE)
(72) Erfinder: Meyer, Oliver, Dr., 48151 Münster (DE); Kalz, Thomas, 44625 Herne (DE); Drauz, Karlheinz, Prof., 62579 Freigericht (DE)

(56) Entgegenhaltungen:
- DE-B- 2 854 627
- EFFENBERGER F & DRAUZ K: "Neue Synthese von Aminosäuren aus Halogencarbonsäureestern" ANGEWANDTE CHEMIE, Bd. 91, Nr. 6, 1979, Seiten 504-505, XP009007084
- EFFENBERGER F ET AL.: "Darstellung von Aminosäuren aus Halogencarbonsäuren-alkyl-estern mit Alkalimetallcyanaten" CHEMISCHE BERICHTE, Bd. 114, Nr. 1, 1981, Seiten 173-189, XP009007085

## Beschreibung

Die vorliegende Erfindung befaßt sich mit der Herstellung von Aminocarbonsäuren. Insbesondere wird ein Weg aufgezeigt, wie Halogencarbonsäureester mit einem Metallcyanat und einem Alkohol vorteilhaft zu Urethancarbonsäureestern umgesetzt werden können, aus welchen anschließend durch Verseifung die Aminocarbonsäuren freizusetzen sind.

Die im Rahmen dieses Patents beschriebenen Aminocarbonsäuren sind von großer Bedeutung u.a. im Bereich der parenteralen oder Tierernährung, der organischen Synthese z.B. in der Polymerchemie und als Synthesebaustein für die Darstellung von Pharmazeutika.

Es gibt eine Vielzahl von Synthesemethoden zur Herstellung von Aminocarbonsäuren, die dem Fachmann wohlbekannt sind. Zur Herstellung von z.B. ω-Aminocarbonsäuren sind aufgrund ihrer Bedeutung insbesondere im Bereich der Polymerchemie in der Literatur zahlreiche Synthesen aufgezeigt.
So kann z.B. die ω-Aminooctansäure durch Beckmann-Umlagerung mit anschließender Ringöffnung ausgehend von Cyclooctanon hergestellt werden (WO 9850341, Synth. Commun. 2001, 31, 2047-2050; Synth. Commun. 1998, 28, 2275-2280; Synth. Commun. 1996, 16, 2641-2649; J. Chem. Soc., Chem. Commun. 1995, 1101; Synthesis 1979, 537-538; Can. J. Chem. 1992, 70, 186-196). Neben weiteren Beckmann-Umlagerungen, die von längerkettigen aliphatischen Oximen ausgehen (Chem. Ber. 1896, 29, 806-809; Chem. Ber. 1894, 27, 3121-3129; J. Chem. Soc. 1914, 105, 2809; J. Chem. Soc. 1926, 2207), kann ω-Aminooctansäure auch durch halbseitigen Hoffmann-Abbau von Azelainsäure (Chem. Ber. 1956, 89, 117-120) oder durch Curtius-Umlagerung eines Azelainsäuremonoalkylesters synthetisiert werden (Chem. Pharm. Bull. 1959, 7, 99-100). Auch die Synthese durch Hydrierung von 7-Cyanheptansäure (Izv. Akad. Nauk. SSSR Ser. Khim 1955, 224-229, engl. Ausgabe 199-205) oder 7-Cyan-2,5-heptadiensäure (DE 1081469; Angew. Chem. 1960, 72, 74-76) ist in der Literatur beschrieben. Des weiteren findet sich in der Literatur die Synthese der ω-Aminooctansäure durch Umsetzung von 8,8-Dichlor-7-octenylamin mit wässriger Schwefelsäure (Zh. Obshch. Khim. 1957, 27, 2418-2421, engl. Ausgabe 2481-2484) oder durch Reaktion der entsprechenden ω-Chlorcarbonsäure mit flüssigem Ammoniak (FR 1346045).

Die Nachteile der oben aufgezählten Synthesen liegen entweder in der schlechten Zugänglichkeit der Edukte, in den geringen Reaktionsausbeuten oder aber in der mangelnden Durchführbarkeit im großtechnischen Maßstab.

In der Literatur ist die Umsetzung von Halogencarbonsäureestern mit Alkalimetallcyanaten in Gegenwart eines Alkohols und anschließende doppelte Verseifung des gebildeten Urethancarbonsäureesters zur Aminocarbonsäure vorgeschlagen worden (DE 2854627; Angew. Chem. 1979, 91, 504-505; Chem. Ber. 1981, 114, 173-189). Hier berichten die Autoren von der Synthese verschiedener Aminocarbonsäuren durch die Umsetzung von Brom- bzw. Chlorsubstituierten Carbonsäureestern in polar aprotischen Lösemitteln mit Alkalimetallcyanat in Gegenwart eines Alkohols zum Urethan mit anschließender saurer Hydrolyse. Allerdings wird nur der Einsatz vergleichsweise kurzkettiger Halogencarbonsäureester mit einer Kettenlänge von maximalen sechs Kohlenstoffatomen beschrieben. Über die Synthese von höherkettigen Derivaten wird nicht berichtet. Darüber hinaus wird deutlich, dass nach dem beschriebenen Verfahren für die Umsetzung von Chlorcarbonsäureestern zum Urethan wesentlich längere Reaktionszeiten erforderlich sind, als für die entsprechenden Bromcarbonsäureester. So zeigt beispielsweise der Vergleich, dass 6-Chlorhexansäuremethylester erst nach 30 Stunden Reaktionszeit einen vollständigen Umsatz zum 6-(Methoxycarbonylamino)-hexansäuremethylester erreicht, während 6-Bromhexansäuremethylester unter gleichen Reaktionsbedingungen bereits nach 0,75 Stunden vollständig umgesetzt ist. Aufgrund der Tatsache, dass Chlorverbindungen in der technischen Synthese den entsprechenden Bromverbindungen vorzuziehen sind, ist die aufgezeigte Synthesevariante für den großtechnischen Einsatz daher nachteilig.
Auch die hydrolytische Spaltung der Urethangruppe, die mit einem Gemisch aus Ameisensäure und Salzsäure in Wasser beschrieben wird und laut Autoren ein 24 stündiges Erhitzen auf Rückflusstemperatur erfordert, lässt diese Verfahrensweise für den großtechnischen Einsatz als nicht geeignet erscheinen.

Aufgabe der vorliegenden Erfindung war daher die Angabe eines weiteren Verfahrens zur Herstellung von Aminocarbonsäuren anzugeben, welches insbesondere im großtechnischen Maßstab unter ökonomischen und ökologischen Gesichtspunkten betrachtet vorteilhaften anzuwenden ist.

Diese und andere sich aus dem Stand der Technik in naheliegender Weise ergebende Aufgaben werden durch eine Verfahren mit den Merkmalen des Anspruchs 1 gelöst. Die abhängigen Ansprüche 2 bis 10 richten sich auf bevorzugte Ausführungsformen des erfindungsgemäßen Verfahrens.

Dadurch, dass man in einem Verfahren zur Herstellung von Aminocarbonsäuren ausgehend von einem Halogencarbonsäureester durch Umsetzung mit einem Metallcyanat und einem Alkohol und anschließender saurer Verseifung so vorgeht, dass man das Metallcyanat in einem aprotisch-polaren organischen Lösungsmittel bei erhöhten Temperaturen vorlegt und anschließend den Alkohol und den Ester bei erhöhter Temperatur zur Cyanat-Lösung kontinuierlich zudosiert, gelangt man in gegenüber den Verfahren des Standes der Technik überraschender dafür aber nicht minder vorteilhaften Weise in höheren Raum/Zeitausbeuten zu den gewünschten Aminocarbonsäuren.

Vorzugsweise erfindungsgemäß umsetzen lassen sich Halogencarbonsäureester der folgenden allgemeinen Formel (I) 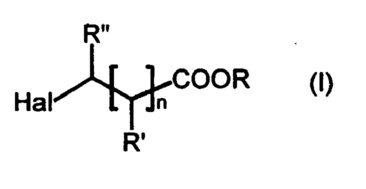 worin
n eine ganze Zahl von 0 bis 10 ist,
R ein (C₁-C₈)-Alkyl ist,
R' und R" unabhängig voneinander für H, (C₁-C₈)-Alkyl, (C₃₋C₈)-Cycloalkyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl)₁₋₃-(C₃₋C₈)-Cycloalkyl, ((C₁-C₈)-Alkyl)₁₋₃-(C₆-C₁₈)-Aryl, ((C₁-C₈)-Alkyl)₁₋₃-(C₃-C₁₈)-Heteroaryl stehen,
Hal Chlor oder Brom bedeutet.

Ebenfalls gut umzusetzen sind Halogendicarbonsäurediester der allgemeinen Formel (II) 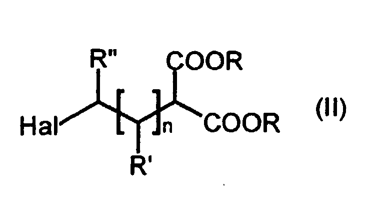 worin
n eine ganze Zahl von 0 bis 10 ist,
R ein (C₁-C₈)-Alkyl ist,
R' und R" unabhängig voneinander für H, (C₁-C₈)-Alkyl, (C₁₋C₈)-Acyl, (C₃-C₈)-Cycloalkyl, (C₆-C₁₈)-Aryl, (C₇-C₁₉)-Aralkyl, (C₃-C₁₈)-Heteroaryl, (C₄-C₁₉)-Heteroaralkyl, ((C₁-C₈)-Alkyl)₁₋₃₋(C₃-C₈)-Cycloalkyl, ((C₁-C₈-Alkyl)₁₋₃-(C₆-C₁₈)-Aryl, ((Ci-C₈)-Alkyl)₁₋₃-(C₃-C₁₈)-Heteroaryl stehen,
Hal Chlor oder Brom bedeutet.

Ganz besonders bevorzugt wird das erfindungsgemäße Verfahren zur Herstellung von ω-Aminocarbonsäuren wie beispielsweise das in der Polymerchemie benötigte ω-Aminooctansäure angewandt.

Als Cyanate können prinzipiell alle dem Fachmann für diesen Zweck in Frage kommenden Verbindungen genommen werden. Die Auswahl richtet sich nach der besten Umsetzbarkeit gekoppelt mit dem niedrigsten Bezugspreis und Gefahrenpotential. Vorzugsweise werden Alkalicyanate als Metallcyanat, besonders bevorzugt Lithium-, Natrium- oder Kaliumcyanat eingesetzt.

Als Alkohole finden dem Fachmann für diesen Zweck naheliegende Verbindungen Anwendung. Der eingesetzte Alkohol sollte möglichst kostengünstig sein und doch die Urethanbildung in geeigneter Manier zulassen. Bevorzugt ist der Einsatz von linearen oder beliebig verzweigten Alkylalkoholen wie z.B. Methanol, Ethanol, Propanol, Isopropanol, Butanol, sec-Butanol, Isobutanol, tert.-Butanol usw.

Das einzusetzende Lösungsmittel bei der Urethanbildung sollte ein aprotisch polares organisches sein. Es sollte eine ausreichende Löslichkeit für die eingesetzten Reaktionspartner besitzen und sich zum anderen bei den Reaktionsbedingungen als inert verhalten. Vorteilhaft sind Lösungsmittel wie Dimethylformamid, Dimethylsulfoxid, n-Methylpyrrolidon oder Acetonitril. Ganz besonders bevorzugt ist Dimethylformamid.

Die Temperatur bei der Urethanbildung ist so zu wählen, daß einerseits die Reaktion möglichst schnell verläuft, andererseits jedoch die Nebenproduktbildung möglichst unterdrückt wird, um eine optimale Raum/Zeitausbeute zu gewährleisten. Durch Routinexperimente kann für die herzustellenden Aminocarbonsäuren die geeignete Temperatur ermittelt werden. Bevorzugt ist die Ausführungsform, bei der die Zugabe des Alkohols und des Halogencarbonsäureesters bei 80°C bis 200°C, vorzugsweise bei 120°C bis 160°C, äußerst bevorzugt bei 140°C durchgeführt wird.

Das Mol-Verhältnis der einzelnen Reaktionspartner zueinander sollte mindestens 1:1:1 für den Halgencarbonsäureester, den Alkohol und das Metallcyanat betragen. Es richtet sich jedoch ansonsten nach den Maßstäben einer optimalen Ausbeute an Produkt in einer möglichst kurzen Zeit in kostengünstiger Weise produzieren zu können. Aus diesem Grund kann vom o.a. Verhältnis abgewichen werden. Vorteilhaft ist der Einsatz eines Überschusses an Alkohol und ggf. Metallcyanat im Bereich von 1:1-2:1-1,5 bezogen auf den Halogencarbonsäureester. Ganz besonders bevorzugt ist die Verwendung eines Überschusses an Alkohol von 10-50 Mol-% bei einem Verhältnis von Halogencarbonsäureester zu Metallcyanat von 1:1.

Die gebildete Urethancarbonsäure kann abschließend zur Aminocarbonsäure verseift werden, wobei im Falle des Einsatzes von Verbindungen der allgemeinen Formel (I) die Verseifung des Esters und des Urethans sowie die Decarboxylierung der resultierenden Carbaminsäurefunktion parallel ablaufen. Im Falle des Einsatzes von Verbindungen der allgemeinen Formal (II) findet darüber hinaus noch eine weitere Verseifung und Decarboxylierung des Malonesters statt. Anhand der frei werdenden Gasmenge kann die Reaktion gut verfolgt werden. Diese Reaktionen können nach dem Fachmann bekannten Verfahren durchgeführt werden. Vorzugsweise wird man die saure Verseifung des gebildeten Urethancarbonsäureesters mittels einer wässrigen Lösung einer Mineralsäure durchführen. Ganz besonders bevorzugt ist der Einsatz von wässriger Salzsäure für diesen Zweck, da die dabei gebildeten Hydrochloride der Aminosäuren als Salze gut durch Kristallisation rein gewonnen werden können.

Für das Erlangen einer hohen Raum/Zeitausbeute scheint es wichtig zu sein, die erfindungsgemäße Zugabereihenfolge der Reaktionspartner in Kombination mit den vorliegenden Reaktionsbedingungen einzuhalten. So sollte der Alkohol zusammen mit dem Halogencarbonsäureester bei erhöhter Temperatur kontinuierlich zu dem vorgelegten Metallcyanat zugegeben werden. Die kontinuierliche Zugabe sorgt dafür, daß die Reaktionspartner Alkohol und Halogencarbonsäureester über fast die gesamte Reaktionsdauer im Unterschuß vorliegen, was offensichtlich einen positiven Einfluß auf die Schnelligkeit und Ausbeute der Produktbildung hat (im Falle von ω-Aminooctansäure 80% innerhalb von 3 Stunden Reaktionsdauer).
Die Kombination der genannten Maßnahmen wird durch den Stand der Technik weder vorweggenommen noch nahegelegt.

Die praktische Umsetzung der Halogencarbonsäureester durch N-Alkylierung zum Urethan erfolgt nach dem erfindungsgemäßen Verfahren also dadurch, dass z.B. ein Alkalimetallcyanat in einem möglichst hochsiedenden polar aprotischen Lösungsmittel vorgelegt und zunächst auf Reaktionstemperatur erhitzt wird. Als Alkalimetallcyanate können dabei Lithium, Natrium- oder Kaliumcyanat verwendet werden, wobei Kaliumcyanat bevorzugt ist. Als Lösemittel geeignet sind insbesondere Dimethylformamid, Dimethylsulfoxid und N-Methylpyrrolidon, wobei Dimethylformamid bevorzugt eingesetzt wird. Die Reaktion wird in Abhängigkeit vom eingesetzten Lösemittel und den Reaktionspartnern bei einer Temperatur von größer 80°C, vorzugsweise bei 140 °C durchgeführt.

Im Anschluss daran wird eine Mischung aus Halogencarbonsäureester und Alkohol kontinuierlich zudosiert und das Reaktionsgemisch bis zum vollständigen Umsatz des Halogencarbonsäureesters auf Reaktionstemperatur gehalten. Im Gegensatz zu literaturbekannten Reaktionen erweist sich ein Überschuss an Metallcyanat beim erfindungsgemäßen Verfahren als überflüssig, so dass später weniger Abfallsalz anfällt. Die Zudosierung von Alkohol und Alkylierungsmittel erfolgt über den gesamten Reaktionszeitraum, vorzugsweise in einem Zeitraum von 0,5 bis 4 Stunden, vorteilhaft innerhalb von 1,5 Stunden.

Durch die geänderte Reaktionsführung erreicht man z. B. bei der Reaktion von Kaliumcyanat mit 8-Chloroctansäureethylester und Ethanol in Dimethylformamid bereits nach 4 Stunden einen quantitativen Umsatz zum Urethan. Dieser Effekt war nicht vorauszusehen und stellt im Vergleich zu den bislang bekannten Umsetzungen Chlorsubstituierter Carbonsäureester eine deutliche Verfahrensverbesserung dar. Ein zusätzlicher Vorteil des erfindungsgemäßen Verfahrens ist, dass es auch auf längerkettige ω-Chlorcarbonsäureester angewandt werden kann.

In einer besonders vorteilhaften Verfahrensweise werden z.B. (6-Chlorhexyl)-malonsäurediester mit Metallcyanat und Alkohol zum entsprechenden Urethan umgesetzt. Diese Verfahrensweise hat den Vorteil, dass bei der sich anschließenden Umsetzung der 6-[(Alkoxycarbonyl)amino]-hexylmalonsäureester mit wässriger Salzsäure in einem einzigen Reaktionsschritt drei Hydrolysen und zwei Decarboxylierungen erfolgen. Auf diese Weise werden eine Vielzahl von Reinigungs- und Isolierungsschritte vermieden, so dass sich das erfindungsgemäße Verfahren besonders für die großtechnische Anwendung eignet.

Das bei der Reaktion anfallende Metallchloridsalz (z.B. Kaliumchlorid) wird durch Filtration abgetrennt. Das eingesetzte Lösemittel wird destillativ vom Produkt abgetrennt und kann erneut in die Reaktion eingesetzt werden. Nach dem erfindungsgemäßen Verfahren können die gebildeten Urethane in roher Form oder nach destillativer Reinigung eingesetzt werden. Vorteilhaft ist dabei der Einsatz der Rohprodukte.

Sofern auf eine Recyclierung des organischen Lösungsmittels verzichtet werden kann, kann die Verseifung auch ohne vorherige destillative Abtrennung des organischen Lösungsmittels gearbeitet werden.

Die Hydrolyse der durch N-Alkylierung hergestellten Urethane erfolgt nach dem erfindungsgemäßen Verfahren dadurch, das die Edukte möglichst ohne Lösemittel vorgelegt und auf eine Reaktionstemperatur von etwa 80-120 °C erhitzt werden. Im Anschluss daran wird z.B. wässrige Salzsäure zudosiert und das Reaktionsgemisch weiter auf 100 °C erhitzt. Die wässrige Salzsäure wird im 4-8fachen Überschuss und in einer Konzentration von 10-37 % eingesetzt, vorteilhaft ist ein 6facher Überschuss und eine Konzentration von 15-20 %. Die Zudosierung der Salzsäure erfolgt über einen Zeitraum von 0,5 bis 3 Stunden, vorteilhaft innerhalb von 1,5 Stunden. Bei der Hydrolyse wird in einem Schritt sowohl die Urethan- als auch die Estergruppe gespalten. Als Abgas entsteht Kohlendioxid. Nach vollständigem Reaktionsumsatz, der anhand der Abgasbildung verfolgt werden kann, wird vorhandenes Wasser z.B. mit Hilfe eines Schleppmittels ausgekreist, wobei auch überschüssige Salzsäure mit entfernt wird. Als Schleppmittel sind insbesondere Cyclohexan, Ethylcyclohexan oder Toluol geeignet, wobei vorteilhafter Cyclohexan eingesetzt wird. Während der Wasserauskreisung fällt das Produkt als gut filtrierbarer Feststoff aus. Die Isolierung des Produktes erfolgt durch Filtration und anschließende Wäsche. Alternativ kann die wässrige Lösung der aminocarbonsäure aber auch durch an sich bekannte Ionenaustauscherverfahren aufgearbeitet werden.

Wird wie oben bereits beschrieben ein (6-Chlorhexyl)-malonsäurediester zum Urethan umgesetzt, erhält man nach Hydrolyse, Wasserauskreisung und Abtrennung des Schleppmittels zunächst die nicht-decarboxylierte (6-Aminohexyl)-malonsäure. Beim Erhitzen dieser Verbindung in Substanz oder in einem hochsiedenden Lösemittel auf über 130-150 °C fällt nach erneuter Decarboxylierung das gewünschte 8-Aminooctansäurehydrochlorid als Feststoff aus.

Im Gegensatz zu den oben genannten Literaturstellen erfolgt bei dem erfindungsgemäßen Verfahren die Hydrolyse der Urethane ohne den Zusatz von Ameisensäure. Durch die geänderte Reaktionsführung wird eine vollständige Umsatz im Falle der Herstellung von ω-Aminocarbonsäuren nicht erst nach 24 Stunden sondern bereits nach 3 Stunden erreicht.

Das vorgestellte Verfahren erlaubt es also in überraschend einfacher Weise auch bei durch Chlor substituierten Carbonsäuren einen Austausch des Chloratoms gegen eine Stickstoff tragende Gruppe in einer für einen großtechnischen Prozeß annehmbaren Zeitspanne und Ausbeute herzustellen.

Es versteht sich von selbst, daß mittels dieser Methode auch Racemate von chiralen Aminocarbonsäuren gewonnen werden können, sofern durch die Reaktion ein Stereozentrum neu aufgebaut wird. Das Racemat kann dann in dem Fachmann bekannter Manier in die einzelnen Enantiomere aufgespalten werden.

Als (C₁-C₈)-Alkyl sind anzusehen Methyl, Ethyl, *n*-Propyl, Isopropyl, *n*-Butyl, Isobutyl, *sec*-Butyl, *tert*-Butyl, Pentyl, Hexyl, Heptyl oder Octyl samt aller Bindungsisomeren. Die Reste können einfach oder mehrfach mit Heteroatomen wie N, P, S, O substituiert oder unterbrochen sein.

Unter einem (C₆-C₁₈)-Arylrest wird ein aromatischer Rest mit 6 bis 18 C-Atomen verstanden. Insbesondere zählen hierzu Verbindungen wie Phenyl-, Naphthyl-, Anthryl-, Phenanthryl-, Biphenylreste. Diese können einfach oder mehrfach mit (C₁-C₈)-Alkoxy, (C₁-C₈)-Haloalkyl, OH, Cl, NH₂, NO₂ substituiert sein. Außerdem kann der Rest ein oder mehrere Heteroatome wie N, O, S aufweisen. (C₁-C₈)-Alkoxy ist ein über ein Sauerstoffatom an das betrachtete Molekül gebundener (C₁-C₈)-Alkyl-Rest.

Ein (C₇-C₁₉)-Aralkylrest ist ein über einen (C₁-C₈)-Alkylrest an das Molekül gebundener (C₆-C₁₈)-Arylrest.

(C₁-C₈)-Haloalkyl ist ein mit einem oder mehreren Halogenatomen substituierter (C₁-C₈)-Alkyl-Rest. Als Halogenatome kommen insbesondere Chlor und Fluor in Betracht.

Ein (C₃-C₁₈)-Heteroarylrest bezeichnet im Rahmen der Erfindung ein fünf-, sechs- oder siebengliedriges aromatisches Ringsystem aus 3 bis 18 C-Atomen, welches Heteroatome wie z. B. Stickstoff, Sauerstoff oder Schwefel im Ring aufweist. Als solche Heteroaromaten werden insbesondere Reste angesehen, wie 1-, 2-, 3-Furyl, wie 1-, 2-, 3-Pyrrolyl, 1-,2-,3-Thienyl, 2-, 3-, 4-Pyridyl, 2-, 3-, 4-, 5-, 6-, 7-Indolyl, 3-, 4-, 5-Pyrazolyl, 2-,4-, 5-Imidazolyl, Acridinyl, Chinolinyl, Phenanthridinyl, 2-, 4-, 5-, 6-Pyrimidinyl. Diese können einfach oder mehrfach mit (C₁-C₈)-Alkoxy, (C₁-C₈)-Haloalkyl, OH, Halogen, NH₂, NO₂, SH, S-(C₁-C₈)-Alkyl substituiert sein.

Unter einem (C₄-C₁₉)-Heteroaralkyl wird ein dem (C₇-C₁₉)-Aralkylrest entsprechendes heteroaromatisches System verstanden.

Unter (C₃-C₈)-Cycloalkyl versteht man Cyclopropyl, Cyclobutyl, Cyclopentyl, Cyclohexyl bzw. Cycloheptyl oder Cyclooctylreste.

Halogen ist Fluor, Chlor, Brom, Iod.

Die dargestellten chemischen Strukuren beziehen sich auf alle möglichen Stereoisomeren, die durch Abänderung der Konfiguration der einzelnen chiralen Zentren, Achsen oder Ebenen erreicht werden können, also alle möglichen Diastereomere, sowie alle darunter fallenden optischen Isomere (Enantiomere).

### Beipiel:

1,5 mol Kaliumcyanat werden in 750 ml Dimethylformamid vorgelegt und auf 140 °C erhitzt. Im Anschluss daran wird eine Mischung aus 1,5 mol 8-Chloroctansäureethylester und 1,65 mol Ethanol über einen Zeitraum von 1 Stunde zudosiert und weitere 3 Stunden bis zum vollständigen Umsatz bei 140 °C gerührt. Das ausgefallene Kaliumchlorid wird abfiltriert und überschüssiges Dimethylformamid am Rotationsverdampfer im Vakuum entfernt. Das so erhaltene Rohprodukt wird ohne weitere Reinigung und ohne Lösemittel bei 100 °C vorgelegt. Dann wird über einen Zeitraum von 1,5 Stunden eine Mischung aus 887 g konz. HCl und 162 g Wasser zudosiert. Nach vollständigem Umsatz (ca. 3 Stunden) wird Cyclohexan zugegeben, sämtliches Wasser ausgekreist und der ausgefallene Feststoff abfiltriert. Nach mehrmaliger Wäsche und Trocknung des Produktes im Vakuum erhält man das Hydrochlorid der 8-Aminooctansäure als weißen, kristallinen Feststoff (Schmelzpunkt: 141-144 °C) mit 80 % Ausbeute.

## Patentansprüche

1. Verfahren zur Herstellung von Aminocarbonsäuren ausgehend von einem Halogencarbonsäureester durch Umsetzung mit einem Metallcyanat und einem Alkohol und anschließender saurer Verseifung,
**dadurch gekennzeichnet, daß**
man das Metallcyanat in einem aprotisch-polaren organischen Lösungsmittel bei erhöhten Temperaturen vorlegt und anschließend den Alkohol und den Ester bei erhöhter Temperatur zur Cyanat-Lösung kontinuierlich zudosiert.

2. Verfahren nach Anspruch 1,
**dadurch gekennzeichnet, daß**
man Halogencarbonsäureester der folgenden allgemeinen Formel (I) 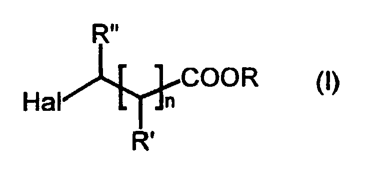 worin
n eine ganze Zahl von 0 bis 10 ist,
R ein (C₁-C₈)-Alkyl ist,
R' und R" unabhängig voneinander für H, (C₁-C₈)-Alkyl, (C₃-C₈)-Cycloalkyl, (C₆-C₁₈)-Aryl, (C₇-C₁₉)-Aralkyl, (C₃-C₁₈)-Heteroaryl, (C₄-C₁₉)-Heteroaralkyl, ((C₁-C₈)-Alkyl)₁₋₃-(C₃-C₈)-Cycloalkyl, ((C₁-C₈)-Alkyl)₁₋₃₋(C₆-C₁₈)-Aryl, ((C₁-C₈)-Alkyl)₁₋₃-(C₃-C₁₈)-Heteroaryl stehen,
Hal Chlor oder Brom bedeutet,
umsetzt.

3. Verfahren nach einem oder mehreren der vorhergehenden Ansprüche,
**dadurch gekennzeichnet, daß**
man Halogendicarbonsäurediester der allgemeinen Formel (II) 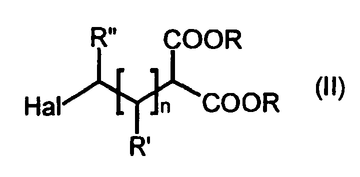 worin
n eine ganze Zahl von 0 bis 10 ist,
R ein (C₁-C₈)-Alkyl ist,
R' und R" unabhängig voneinander für H, (C₁-C₈)-Alkyl, (C₁-C₈)-Acyl, (C₃-C₈)-Cycloalkyl, (C₆-C₁₈)-Aryl, (C₇-C₁₉)-Aralkyl, (C₃-C₁₈)-Heteroaryl, (C₄-C₁₉)-Heteroaralkyl, ((C₁-C₈)-Alkyl)₁₋₃-(C₃-C₈)-Cycloalkyl, ((C₁-C₈)-Alkyl)₁₋₃-(C₆-C₁₈)-Aryl, ((C₁-C₈)-Alkyl)₁₋₃-(C₃₋C₁₈)-Heteroaryl stehen,
Hal Chlor oder Brom bedeutet,
umsetzt.

4. Verfahren nach einem oder mehreren der vorhergehenden Ansprüche,
**dadurch gekennzeichnet, daß**
man ω-Aminocarbonsäuren herstellt.

5. Verfahren nach einem oder mehreren der vorhergehenden Ansprüche,
**dadurch gekennzeichnet, daß**
man als Metallcyanat Lithium-, Natrium- oder Kaliumcyanat verwendet.

6. Verfahren nach einem oder mehreren der vorhergehenden Ansprüche,
**dadurch gekennzeichnet, daß**
man als Alkohol einen Alkylalkohol wie Methanol oder Ethanol einsetzt.

7. Verfahren nach einem oder mehreren der vorhergehenden Ansprüche,
**dadurch gekennzeichnet, daß**
man als aprotisch-polares organisches Lösungsmittel Dimethylformamid, Dimethylsulfoxid, n-Methylpyrrolidon oder Acetonitril.

8. Verfahren nach einem oder mehreren der vorhergehenden Ansprüche,
**dadurch gekennzeichnet, daß**
man die Zugabe des Alkohols und des Halogencarbonsäureesters bei 120°C bis 160°C durchführt.

9. Verfahren nach einem oder mehreren der vorhergehenden Ansprüche,
**dadurch gekennzeichnet, daß**
man bezogen auf den Halogencarbonsäureester den Alkohol und das Metallcyanat im Mol-Verhältnis 1:1-2:1-1,5 einsetzt.

10. Verfahren nach einem oder mehreren der vorhergehenden Ansprüche,
**dadurch gekennzeichnet, daß**
man die saure Verseifung des gebildeten Urethancarbonsäureesters mittels einer wässrigen Lösung einer Mineralsäure durchführt.

## Claims

1. A method of producing an amino carboxylic acid from a halo carboxylic ester by reaction with a metal cyanate and an alcohol and subsequent acidic saponification, **characterized in that** the metal cyanate is provided as an initial charge in an aprotic polar organic solvent at an elevated temperature and then the alcohol and the ester are continuously metered into the cyanate solution at elevated temperature.

2. A method according to claim 1, **characterized in that** a halo carboxylic ester of the following general formula (I): 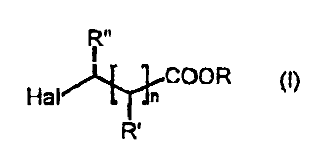 where
n is a whole number from 0 to 10,
R is (C₁-C₈)-alkyl,
R' and R" are independently H, (C₁-C₈)-alkyl, (C₃-C₈)-cycloalkyl, (C₆-C₁₈)-aryl, (C₇-C₁₉)-aralkyl, (C₃-C₁₆)-heteroaryl, (C₄-C₁₉)-heteroaralkyl, ((C₁-C₈)-alkyl)₁₋₃-(C₃-C₈)-cycloalkyl, ((C₁-C₈)-alkyl)₁₋₃-(C₆-C₁₈)-aryl, ((C₁-C₈)-alkyl)₁₋₃-(C₃-C₁₈)-heteroaryl, and
Hal is chlorine or bromine,
is reacted.

3. A method according to one or more of the preceding claims, **characterized in that** a halo dicarboxylic diester of the general formula (II) 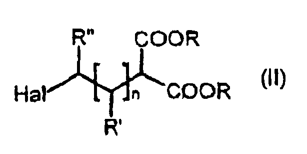 where
n is a whole number from 0 to 10,
R is (C₁-C₈)-alkyl,
R' and R" are independently H, (C₁-C₈)-alkyl, (C₁₋C₈)-acyl, (C₃-C₈)-cycloalkyl, (C₆-C₁₈)-aryl, (C₇-C₁₉)-aralkyl, (C₃-C₁₈)-heteroaryl, (C₄-C₁₉)-heteroaralkyl, ((C₁-C₈)-alkyl)₁₋₃-(C₃-C₈)-cycloalkyl, ((C₁-C₈)-alkyl)₁₋₃-(C₆-C₁₈)-aryl, ((C₁-C₈)-alkyl)₁₋₃₋(C₃-C₁₈)-heteroaryl, and
Hal is chlorine or bromine,
is reacted.

4. A method according to one or more of the preceding claims, **characterized in that** an ω-amino carboxylic acid is produced.

5. A method according to one or more of the preceding claims, **characterized in that** lithium cyanate, sodium cyanate or potassium cyanate is used as metal cyanate.

6. A method according to one or more of the preceding claims, **characterized in that** an alkyl alcohol such as methanol or ethanol is used as alcohol.

7. A method according to one or more of the preceding claims, **characterized in that** dimethylformamide, dimethyl sulphoxide, N-methylpyrrolidone or acetonitrile is used as aprotic polar organic solvent.

8. A method according to one or more of the preceding claims, **characterized in that** the alcohol and the halocarboxylic ester are added at a temperature in the range from 120°C to 160°C.

9. A method according to one or more of the preceding claims, **characterized in that** the halo carboxylic ester, the alcohol and the metal cyanate are used in a molar ratio of 1:1-2:1-1.5.

10. A method according to one or more of the preceding claims, **characterized in that** the acidic saponification of the urethane carboxylic ester formed is effected by means of an aqueous solution of a mineral acid.

## Revendications

1. Procédé pour préparer des acides amino-carboxyliques à partir d'un ester d'acide carboxylique halogéné, par réaction avec un cyanate métallique et un alcool et ensuite par saponification acide,
**caractérisé en ce qu'**
on place le cyanate métallique dans un solvant organique polaire aprotique à hautes températures et on dose ensuite en continu l'alcool et l'ester à haute température pour obtenir une solution de cyanate.

2. Procédé selon la revendication 1,
**caractérisé en ce qu'**
on fait réagir l'ester d'acide carboxylique halogéné de formule générale (I) suivante : 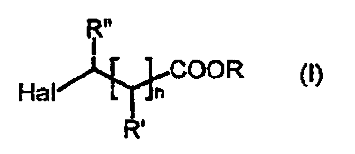 dans laquelle :
n est un entier valant 0 à 10,
R est un groupe alkyle en C₁-C₈,
R' et R", indépendamment l'un de l'autre, sont H, un groupe alkyle en C₁-C₈, un groupe cycloalkyle en C₃-C₈, un groupe aryle en C₆-C₁₈, un groupe aralkyle en C₇-C₁₉, un groupe hétéroaryle en C₃-C₁₈, un groupe hétéroaralkyle en C₄-C₁₉, un groupe (alkyle en C₁-C₈)₁₋₃-(cycloalkyle en C₃-C₈), un groupe (alkyle en C₁-C₈)₁₋₃-(aryle en C₆-C₁₈), un groupe (alkyle en C₁-C₈)₁₋₃-(hétéroaryle en C₃-C₁₈),
Hal désigne le chlore ou le brome.

3. Procédé selon l'une ou plusieurs des revendications précédentes,
**caractérisé en ce qu'**
on fait réagir du diester d'acide dicarboxylique halogéné de formule générale (I) suivante : 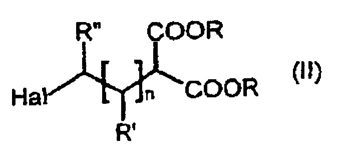 dans laquelle :
n est un entier valant 0 à 10,
R est un groupe alkyle en C₁-C₈,
R' et R", indépendamment l'un de l'autre, sont H, un groupe alkyle en C₁-C₈, un groupe acyle en C₁-C₈, un groupe cycloalkyle en C₃-C₈, un groupe aryle en C₆-C₁₈, un groupe aralkyle en C₇-C₁₉, un groupe hétéroaryle en C₃-C₁₈, un groupe hétéroaralkyle en C₄-C₁₉, un groupe (alkyle en C₁-C₈)₁₋₃-(cycloalkyle en C₃-C₈), un groupe (alkyle en C₁-C₈)₁₋₃-(aryle en C₆-C₁₈), un groupe (alkyle en C₁-C₈)₁₋₃-(hétéroaryle en C₃-C₁₈),
Hal désigne le chlore ou le brome.

4. Procédé selon l'une ou plusieurs des revendications précédentes,
**caractérisé en ce qu'**
on prépare des acides ω-amino-carboxiliques.

5. Procédé selon l'une ou plusieurs des revendications précédentes,
**caractérisé en ce que**
comme cyanate métallique du cyanate de lithium, on utilise du cyanate de sodium ou du cyanate de potassium.

6. Procédé selon l'une ou plusieurs des revendications précédentes,
**caractérisé en ce que**
comme alcool, on utilise un alcool alkylique comme le méthanol ou l'éthanol.

7. Procédé selon l'une ou plusieurs des revendications précédentes,
**caractérisé en ce que**
comme solvant organique polaire aprotique, on utilise du diméthylformamide, du diméthylsulfoxyde, de la n-méthylpyrrolidone ou de l'acétonitrile.

8. Procédé selon l'une ou plusieurs des revendications précédentes,
**caractérisé en ce qu'**
on réalise l'addition de l'alcool et de l'ester d'acide carboxylique halogéné à 120°C-160°C.

9. Procédé selon l'une ou plusieurs des revendications précédentes,
**caractérisé en ce qu'**
on emploie, sur la base de l'ester d'acide carboxylique halogéné, l'alcool et le cyanate métallique selon le rapport molaire de 1 / 1 à 2 / 1-1,5.

10. Procédé selon l'une ou plusieurs des revendications précédentes,
**caractérisé en ce qu'**
on réalise la saponification acide de l'ester d'acide carboxylique d'uréthane formé au moyen d'une solution aqueuse d'un acide minéral.
